# EUROPEAN PATENT SPECIFICATION

(11) **EP 0 505 891 B1**
(45) Date of publication and mention of the grant of the patent: **12.02.2003**
(21) Application number: 92104553.0
(22) Date of filing: 17.03.1992
(51) Int. Cl.: C07D 241/12

(54) **Process for the preparation of alkylpyrazines**
Verfahren zur Herstellung von Alkylpyrazinen
Procédé de préparation de alcoylpyrazines

(30) Priority: 26.03.1991 US 675417
(43) Date of publication of application: 30.09.1992
(73) Proprietor: SOCIETE DES PRODUITS NESTLE S.A., 1800 Vevey (CH)
(72) Inventor: Chen, Teh-Kuei, Gaylordsville, CO 06755 (US)

(56) References cited:
- CHEMICAL ABSTRACTS, vol. 88, no. 15, 10 April 1978, Columbus, Ohio, US; abstract no. 105411E, T. KOSUGE ET AL.: 'Alkylpyrazines.' page 582 ;
- JOURNAL OF AGRICULTURAL AND FOOD CHEMISTRY. vol. 36, no. 2, 1988, WASHINGTON US pages 349 - 352; G.P. RIZZI: 'Formation of Pyrazines from Acyloin Precursors under Mild Conditions.'

## Description

The present invention relates to a process for the preparation of pyrazines, more particularly alkyl pyrazines.

Alkyl pyrazines are important flavour components which are formed during the cooking of many kinds of foods. Generally, they impart roasted or burnt aromas to cooked foods. There is an expectation of an increase in the usage of alkyl pyrazines in the food industry, especially in the booming market of microwavable foods.

The formation of alkyl pyrazines in foods has been extensively studied using carbohydrate-ammonia model systems. But these types of reactions always give a complex mixture of products with low total yields. It has been proposed that the biochemically available acyloins react with ammonia to produce the corresponding alkyl pyrazines in cheese, in fermented cocoa and soya products.

In a recent publication G.P. Rizzi, J. Agric Food Chem.36, 349 (1988) described the formation of alkyl pyrazines from acyloins and ammonium acetate under mild conditions. The best yield of 58% for tetramethyl pyrazine was reported. But the described procedure involved a long reaction time (15.5 hours at 120°C) in a sealed ampule in darkness, and a tedious process to obtain the product: steam distillation, solvent extraction, solvent removal and final vacuum distillation. The yield obtained for dimethylpyrazine was only 2.3%, the reaction being carried out in the dark at 22°C for 247 hours.

We have developed a significantly simplified method compared with the Rizzi procedure, for the preparation of alkyl pyrazines which gives yields as good as or significantly higher than the Rizzi procedure. The reaction can be accomplished in less than 40 minutes under optimal conditions.

Accordingly, the present invention provides a process for the preparation of an alkyl pyrazine which comprises refluxing a mixture of an acyloin having the general formula: 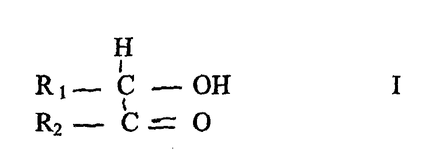 wherein R₁ and R₂ are either both methyl radicals or R₁ is hydrogen and R₂ is a saturated or unsaturated alkyl radical containing from 1 to 10 carbon atoms and an ammonium salt in water and isolating the alkyl pyrazine characterised in that hydrogen peroxide, peroxyacetic acid, peroxybenzoic acid or m-chloro peroxybenzoic acid is added to the reaction mixture.

When R₁ is hydrogen, R₂ preferably contains from 1 to 4 carbon atoms and may be, for example, a methyl, ethyl, n-propyl or isopropyl radical. Most preferably, R₂ is methyl or ethyl. The ammonium salt may be the salt of an organic or inorganic acid, e.g. ammonium citrate, tartrate, formate, acetate or diammonium phosphate. Ammonium acetate is preferred.

The amount of water may vary considerably e.g. from 0.5 to 100 parts by weight per part of the total weight of acyloin and ammonium salt.

The ratio of the acyloin to the ammonium salt may conveniently be a 1:1 to 1:3 molar ratio. Even higher amounts of ammonium salt may be used to speed up the reaction but such higher amounts do not increase the yield. Excess ammonium acetate also serves to buffer the formation of acetic acid during the reaction. Sodium acetate, trisodium phosphate and other alkali salts can also be used to replace ammonium acetate to buffer the pH, if desired, from falling below 5. When the acyloin is acetoin and the ammonium salt is ammonium acetate the resulting product is tetramethyl pyrazine.

In the reflux method the acyloin and ammonium salt are refluxed in the presence of water optionally under pressure. The duration of the reaction is advantageously less than 15 hours and preferably less than 10 hours e.g. from 2 to 8 hours but can be less than 40 minutes under optimal conditions. The isolation of the alkyl pyrazine may be carried out simply by diluting with alkali, e.g. to a pH of at least 8 and separating the crude product e.g. by filtering and crystallising the crude product from hot water.

In the preparation of dialkylpyrazines, it is advantageous to use the method of dropwise addition in the reaction e.g. dropwise addition of the acyloin to the reflux solution and preferably dropwise addition together with simultaneous steam distillation.

If desired, the acyloin and ammonium salt together with a little water may be refluxed with simultaneous sublimation in an apparatus having a wide body and a narrow neck with a large surface area. For example, the apparatus may contain a reducing adaptor between the sublimation body and the sublimation flask, which can catch the sublimed product as it forms and substantialy prevent it falling back into the reaction flask. The reducing adapter may, for instance, have a reduced lower narrow neck, which can catch the sublimed product.

In an advantageous procedure using either the reflux or the sublimation method, the system may be evacuated with a vacuum pump for instance for a few minutes and then closed from the vacuum source before reacting the mixture. To facilitate the sublimation, the pressure under the vacuum may conveniently be from 10 to 100 mm Hg, preferably from 20 to 50 mm Hg. The isolable yield may be increased by this method.

The peroxyacetic acid, peroxybenzoic acid, m.chloro peroxybenzoic acid or hydrogen peroxide are added to the mixture to obtain an improved yield and speed up the reaction, for example in an amount up to 1 molar ratio based on the acyloin, preferably a molar ratio from 0.50 to 0.70 based on the acyloin.

Advantageously an alkaline material may be added to increase the pH above 7, for example sodium acetate or trisodium phosphate.

In our process there is no need to carry out the reaction in a sealed ampule or in darkness: it needs only 40 minutes of reaction under optimal conditions. Also the isolation of our product can be carrried out without the tedious combination of steps including, solvent extraction, solvent removal and final vacuum distillation.

The following Examples further illustrate the present invention.

### Example 1

Acetoin (10.6 g, 0.12 moles) 25 g of ammonium acetate (0.29 moles) and 8 g of 30% H₂O₂ (0.07 moles) in 25 ml of water were heated in an oil bath preheated to 125°C for 40 minutes. After it was cooled to room temperature, the reaction mixture was diluted with 70 ml of water and then the pH was adjusted to 10 with 20% NaOH. The crude product was collected by filtration. Crystallization from hot water yielded 7.3 g of crystalline product (89% yield; m.p. 84-85°C).

### Example 2

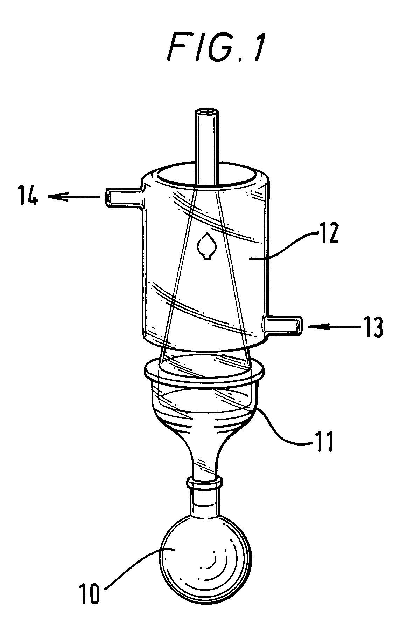

A modified Ace Glass' sublimation apparatus, shown in Fig. 1 of the drawings was used. The apparatus consists of a sublimation flask 10, and adapter 11, and a sublimation body 12 with an inlet 13 and outlet 14.

Acetoin (5.3g), ammonium acetate (6g), sodium acetate (9g), 30% H₂O₂ (4g) in 4ml water were weighed into a 100 ml round bottom flask. The sublimation apparatus was assembled and evacuated to 30 mm Hg for 5 minutes and then closed from the vacuum source. The reaction mixture was heated for 3 hours at 125°C. The crystalline product that sublimed on the cooled wall was collected and washed with water (3.55 g, m.p. 83-85°C). The remaining reaction mixture was diluted with 90 ml of 5% NaOH solution. When filtered, it gave additional crude product.

Crystallization from hot water yielded 0.2 g of extra product (m.p. 83-86°C). The total yield was 3.75 g (91.5% yield).

### Example 3

A modified Ace Glass' sublimation apparatus, shown in Fig. 1 of the drawings was used. The apparatus consists of a sublimation flask 10, an adapter 11, and a sublimation body 12 with an inlet 13 and outlet 14.

Acetoin (5.3 g), ammonium acetate (6g), sodium acetate (5g), trisodium phosphate (4g) and hydrogen peroxide 30% (4 g) in 7 ml of water were weighed into a 100 ml round bottom flask. The sublimation apparatus was assembled and evacuated to 30 mm Hg for 5 minutes and then closed from the vacuum source. The reaction mixture was heated at 90°C for 1 hour and 4 hours at 105°C. The crystalline product that sublimed on the cooled wall was collected and washed with water (3.63 g, m.p. 83-85°C). The remaining reaction mixture was diluted with 90 ml of 5% NaOH solution. When filtered, it gave additional crude product.

Crystallization from hot water yielded 0.2 g of extra product (m.p. 83-86°C). The total yield was 3.83 g (93.4% yield).

### Example 4

Acetoin (5.3 g), ammonium acetate (6 g), sodium acetate (5 g), trisodium phosphate (4 g), and hydrogen peroxide 30% (4 g) in 7 ml of water were weighed into a 100 ml round bottom flask and the whole system was evacuated to 30 mm Hg with a vacuum pump for 5 minutes. The reaction system was then closed from the vacuum source and reacted for 6 hours at an internal temperature of 105°C. The reaction mixture was cooled and treated in the same way as in Example 1. The crystallized product yield was 3.4 g (82.9%, m.p. 83-85°C).

### Example 5

The reaction described in Example 4 was repeated but the reflux was in an open system. The yield was 79.3% (3.25 g, m.p. 83-85°C).

### Example 6

A solution of 60 g ammonium acetate and 20 g of sodium acetate in 200 ml of water was placed in a 3-neck 500 ml round bottom flask. A distillation head was attached for steam distillation. The acetate solution in the flask was heated in an oil bath maintained at 150°C. When the steam distillation began, a solution of 22.3 g of acetol and 20 g of 35% H₂O₂ (0.21 moles) in 250 ml of water was added dropwise at a rate approximately equal to the rate of distillation. The addition was complete in about 4 hours. Then 200 ml of 10% NaOH solution and tap water were added in succession at the same rate. The distillation was continued until about 600 ml of distillate was collected. 60 g of NaCl were added to the distillate which was then extracted with methylene chloride (5x15 ml). The combined extracts were dried over anhydrous Na₂SO₄ and the solvent evaporated. The crude product was purified by vacuum distillation to yield 5.5 g of a mixture of 2,5 and 2,6-dimethylpyrazine (33.7 yield) b.p. 84-88°C (110 mm Hg).

### Example 7

The procedure of Example 6 was followed but using a heated solution of 55 g of ammonium acetate and 30 g of sodium acetate in 200 ml of water to which a solution of 22 mg of 1-hydroxy-2-butanone (0.25 moles) and 18 g of 35% H₂O₂ (0.18 moles) in 250 ml of water was added in carrying out the reaction. The extracted crude product was vacuum distilled to yield 5.3 g of 2,5 and 2,6-diethylpyrazine (31.2% yield) b.p. 113-117°C (110 mm Hg).

## Claims

1. A process for the preparation of an alkyl pyrazine which comprises refluxing a mixture of an acyloin having the general formula: 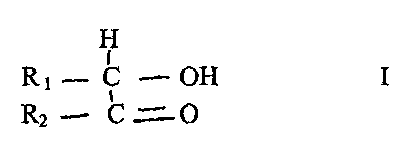 wherein R₁ and R₂ are either both methyl radicals or R₁ is hydrogen and R₂ is a saturated or unsaturated alkyl radical containing from 1 to 10 carbon atoms and an ammonium salt in water and isolating the product **characterised in that** hydrogen peroxide, peroxyacetic acid, peroxybenzoic acid or m-chloro peroxybenzoic acid is added to the reaction mixture.

2. A process according to claim 1 wherein R₁ is hydrogen and R₂ contains from 1 to 4 carbon atoms.

3. A process according to claim 1 wherein R₁ is hydrogen and R₂ is methyl or ethyl.

4. A process according to claim 1 wherein the molar ratio of the acyloin to the ammonium salt is from 1:1 to 1:3.

5. A process according to claim 1 wherein the reaction mixture contains sodium acetate, trisodium phosphate or other alkali salts or excess ammonium acetate to buffer the formation of acetic acid.

6. A process according to claim 1 wherein the refluxing is carried out under pressure.

7. A process according to claim 1 wherein in the preparation of a dialkylpyrazine, the reaction is carried out using the method of dropwise addition.

8. A process according to claim 7 wherein the acyloin is added dropwise to the reaction mixture.

9. A process according to claim 7 wherein the acyloin is added dropwise to the reaction mixture together with simultaneous steam distillation.

10. A process according to claim 1 wherein the refluxing is carried out with simultaneous sublimation in an apparatus having a wide body and a narrow neck with a large surface area.

11. A process according to claim 10 wherein the apparatus contains a reducing adaptor between the sublimation body and the sublimation flask, which can catch the sublimed product as it forms and substantially prevent it falling back into the sublimation flask.

12. A process according to claim 1 wherein the system is evacuated with a vacuum pump and then closed from the vacuum source before reacting the mixture.

13. A process according to claim 1 wherein the pH of the reaction is above 7.

14. A process according to claim 1 wherein the product is isolated by diluting with alkali, separating the crude product and crystallising from hot water.

## Patentansprüche

1. Verfahren zur Herstellung eines Alkylpyrazins, das das Rückflußkochen einer Mischung aus einem Acyloin der allgemeinen Formel 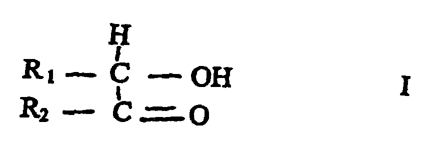 worin R₁ und R₂ entweder beide Methylreste sind oder R₁ Wasserstoff ist und R₂ ein gesättigter oder ungesättigter Alkylrest mit von 1 bis 10 Kohlenstoffatomen ist, und einem Ammoniumsalz in Wasser und das Isolieren des Produkts umfaßt, **dadurch gekennzeichnet, daß** der Reaktionsmischung Wasserstoffperoxid, Peroxyessigsäure, Peroxybenzoesäure oder m-Chlorperoxybenzoesäure zugesetzt wird.

2. Verfahren nach Anspruch 1, bei dem R₁ Wasserstoff ist und R₂ von 1 bis 4 Kohlenstoffatome enthält.

3. Verfahren nach Anspruch 1, wobei R₁ Wasserstoff ist und R₂ Methyl oder Ethyl ist.

4. Verfahren nach Anspruch 1, bei dem das Mol-Verhältnis des Acyloins zu dem Ammoniumsalz von 1:1 bis 1:3 beträgt.

5. Verfahren nach Anspruch 1, bei dem die Reaktionsmischung Natriumacetat, Trinatriumphosphat oder andere Alkalisalze oder einen Überschuß Ammoniumacetat enthält, um die Bildung von Essigsäure abzupuffern.

6. Verfahren nach Anspruch 1, bei dem das Rückflußkochen unter Druck durchgeführt wird.

7. Verfahren nach Anspruch 1, bei dem bei der Herstellung eines Dialkylpyrazins die Reaktion unter Anwendung eines Verfahrens der tropfenweisen Zugabe durchgeführt wird.

8. Verfahren nach Anspruch 7, bei dem das Acyloin tropfenweise zu der Reaktionsmischung zugegeben wird.

9. Verfahren nach Anspruch 7, bei dem das Acyloin zu der Reaktionsmischung tropfenweise unter gleichzeitiger Dampfdestillation zugegeben wird.

10. Verfahren nach Anspruch 1, bei dem das Rückflußkochen unter gleichzeitiger Sublimation in einer Apparatur durchgeführt wird, die einen weiten Körper und einen engen Hals bei einer großen Oberfläche aufweist.

11. Verfahren nach Anspruch 10, bei dem die Apparatur ein Reduzierstück zwischen dem Sublimationskörper und dem Sublimationskolben enthält, das das sublimierte Produkt im Zuge seiner Bildung fängt und es im wesentlichen verhindert, daß es zurück in den Sublimationskolben fällt.

12. Verfahren nach Anspruch 1, bei dem das System mit einer Vakuumpumpe evakuiert und dann von der Vakuumquelle getrennt wird, bevor man die Mischung reagieren läßt.

13. Verfahren nach Anspruch 1, bei dem der pH der Reaktion über 7 liegt.

14. Verfahren nach Anspruch 1, bei dem das Produkt dadurch isoliert wird, daß man es mit Alkali verdünnt, das Rohprodukt abtrennt und aus heißem Wasser kristallisiert.

## Revendications

1. Procédé pour la préparation d'une alkylpyrazine, qui comprend le chauffage au reflux d'un mélange d'une acyloïne répondant à la formule générale : 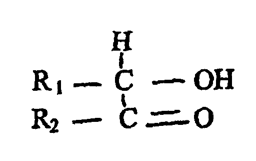 dans laquelle R₁ et R₂ représentent l'un et l'autre un radical méthyle ou bien R₁ représente l'hydrogène et R₂ représente un radical alkyle saturé ou insaturé contenant 1 à 10 atomes de carbone, et d'un sel d'ammonium dans de l'eau, et l'isolement du produit, **caractérisé en ce que** du peroxyde d'hydrogène, de l'acide peroxyacétique, de l'acide peroxybenzoïque ou de l'acide m-chloroperoxybenzoique est ajouté au mélange réactionnel.

2. Procédé suivant la revendication 1, dans lequel R₁ représente l'hydrogène et R₂ contient 1 à 4 atomes de carbone.

3. Procédé suivant la revendication 1, dans lequel R₁ représente l'hydrogène et R₂ représente un groupe méthyle ou éthyle.

4. Procédé suivant la revendication 1, dans lequel le rapport molaire de l'acyloïne au sel d'ammonium est compris dans l'intervalle de 1:1 à 1:3.

5. Procédé suivant la revendication 1, dans lequel le mélange réactionnel contient de l'acétate de sodium, du phosphate trisodique ou d'autres sels de métaux alcalins ou un excès d'acétate d'ammonium pour tamponner la formation d'acide acétique.

6. Procédé suivant la revendication 1, dans lequel le chauffage au reflux est effectué sous pression.

7. Procédé suivant la revendication 1, dans lequel, dans la préparation d'une dialkylpyrazine, la réaction est conduite en utilisant le procédé d'addition goutte à goutte.

8. Procédé suivant la revendication 7, dans lequel l'acyloïne est ajoutée goutte à goutte au mélange réactionnel.

9. Procédé suivant la revendication 7, dans lequel l'acyloïne est ajoutée goutte à goutte au mélange réactionnel conjointement avec une distillation simultanée à la vapeur d'eau.

10. Procédé suivant la revendication 1, dans lequel le chauffage au reflux est effectué avec sublimation simultanée dans un appareil ayant un corps large et un col étroit, avec une grande surface.

11. Procédé suivant la revendication 10, dans lequel l'appareil contient un adaptateur de réduction entre le corps de sublimation et le ballon de sublimation, qui peut piéger le produit sublimé lors de sa formation et l'empêcher substantiellement de retomber dans le ballon de sublimation.

12. Procédé suivant la revendication 1, dans lequel le système est mis sous vide au moyen d'une pompe à vide et est ensuite séparé hermétiquement de la source de vide avant la réaction du mélange.

13. Procédé suivant la revendication 1, dans lequel le pH de la réaction est supérieur à 7.

14. Procédé suivant la revendication 1, dans lequel le produit est isolé par dilution avec un agent alcalin, séparation du produit brut et cristallisation dans l'eau chaude.
